(19) Europäisches Patentamt
European Patent Office
Office européen des brevets

(11) Veröffentlichungsnummer: **0 007 525**
**A1**

(12) # EUROPÄISCHE PATENTANMELDUNG

(21) Anmeldenummer: 79102395.5

(22) Anmeldetag: 12.07.79

(51) Int. Cl.³: C 07 D 401/04
C 07 D 405/14, C 07 D 409/14
A 61 K 31/445, A 61 K 31/47

(30) Priorität: 18.07.78 DE 2831447
15.03.79 DE 2910195

(43) Veröffentlichungstag der Anmeldung:
06.02.80 Patentblatt 80/3

(84) Benannte Vertragsstaaten:
AT BE CH DE FR GB IT NL

(71) Anmelder: HOECHST AKTIENGESELLSCHAFT
Zentrale Patentabteilung Postfach 80 03 20
D-6230 Frankfurt/Main 80(DE)

(72) Erfinder: Lattrell, Ruldolf, Dr.
Heuhohlweg 6H
D-6240 Königstein/Taunus(DE)

(72) Erfinder: Bartmann, Wilhelm, Dr.
Am Dachsbau 5
D-6232 Bad Soden am Taunus(DE)

(72) Erfinder: Kaiser, Joachim, Dr.
Egerländer Strasse 8
D-6233 Kelkheim (Taunus)(DE)

(54) **2-(4-Aminopiperidino)-3,4-dihydrochinoline, Verfahren zu ihrer Herstellung, pharmazeutische Präparate auf Basis dieser Verbindungen sowie ihre Verwendung.**

(57) Verbindungen der Formel

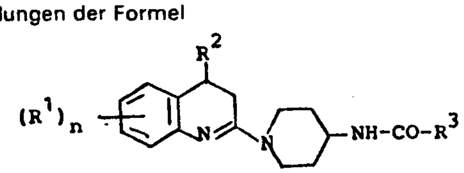

worin n, R¹, R² und R³ die angegebenen Bedeutungen haben, deren physiologisch verträgliche Salze und Verfahren zu ihrer Herstellung.

Die Verbindungen besitzen wertvolle pharmakologische, insbesondere Herz-Kreislauf-wirksame Eigenschaften und sind daher als Arzneimittel geeignet.

EP 0 007 525 A1

HOECHST AKTIENGESELLSCHAFT HOE 78/F 148 K Dr.D/Pa

## 2-(4-Aminopiperidino)-3,4-dihydrochinoline, Verfahren zu ihrer Herstellung, pharmazeutische Präparate auf Basis dieser Verbindungen sowie ihre Verwendung

Die Erfindung betrifft neue 2-(4-Aminopiperidino)-3,4-dihydrochinolinderivate, die wertvolle pharmakologische, insbesondere Herz-Kreislauf-wirksame Eigenschaften besitzen, und daher als Arzneimittel geeignet sind.

Gegenstand der Erfindung sind 2-(4-Aminopiperidino)-3,4-dihydrochinoline der Formel

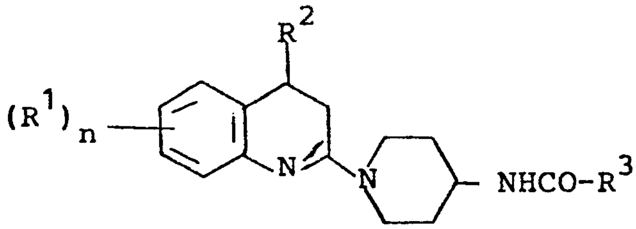

I

worin bedeuten:

n eine Zahl von 1 bis 3

$R^1$ gleiche oder verschiedene Reste der Bedeutung: Wasserstoff $C_1$-$C_6$-Alkyl, $C_1$-$C_6$-Alkoxy, Benzyloxy, Methylendioxy, Halogen, Trifluormethyl, Hydroxy, Nitro, Amino, $C_1$-$C_6$-Alkoxycarbonyl, Sulfamoyl

$R^2$ Wasserstoff, Phenyl, Benzyl, $C_1$-$C_4$-Alkyl und

$R^3$ $C_1$-$C_6$-Alkyl, $C_2$-$C_6$-Alkenyl, $C_1$-$C_6$ Alkyloxy, das gegebenenfalls mit Hydroxy substituiert ist, $C_2$-$C_6$-Alkenyloxy, Cycloalkyl mit 5 bis 7 Kohlenstoffatomen, Cycloalkoxy mit 5 bis 7 Kohlenstoffatomen, Aryl, Aryl-$C_1$-$C_6$-alkyl, Diaryl-$C_1$-$C_6$-alkyl, Aryl-$C_1$-$C_6$-alkyloxy oder Aryloxy, wobei Aryl für Phenyl, Furyl, Thienyl oder Pyridyl steht und wobei die Arylgruppen mono-, di- oder trisubstituiert sein können mit $C_1$-$C_6$-Alkyl, $C_1$-$C_6$-Alkoxy, Benzyloxy, Methylendioxy, Halogen Trifluormethyl, Hydroxy, Nitro, Amino, $C_1$-$C_6$-Alkoxy-carbonyl oder Sulfamoyl,

sowie deren physiologisch verträgliche Salze.

Falls der Arylrest di- oder trisubstituiert ist, können die Substituenten gleich oder verschieden sein.

Als bevorzugte Substituenten kommen in Betracht

für $R^1$: Wasserstoff, Methyl, Äthyl, Methoxy, Äthoxy, Methylendioxy, Chlor, Nitro, Amino

für n: die Zahl 1 oder 2

für $R^2$: Wasserstoff, Phenyl

für $R^3$: geradkettiges oder verzweigtes $C_1$-$C_4$-Alkyl bzw. $C_2$-$C_4$-Alkenyl, geradkettiges oder verzweigtes $C_1$ -$C_4$-Alkyloxy wie Methoxy, Äthoxy, Propyloxy, Isopropyloxy, Isobutyloxy, tert. Butyloxy, mit Hydroxy substituiertes $C_1$-$C_4$-Alkoxy wie z.B. 2-Hydroxy-3-methyl-propyloxy, geradkettiges oder verzweigtes $C_2$-$C_4$-Alkenyloxy wie 2-Methyl-propenyloxy, Cyclohexyl, ein unsubstituierter Phenylrest, oder ein Phenylrest, der mit Methyl, Äthyl, Dimethoxy, Trimethoxy, Methylendioxy, Trifluormethyl, Nitro, Amino, Fluor, Chlor, Brom oder Sulfamoyl substituiert ist oder ein 2- oder 3-Furylrest, ein 2- oder 3-Thienylrest, der durch Chlor und/oder Sulfamoyl substituiert sein kann oder ein 2-, 3- oder 4-Pyridylrest, Benzyl, Diphenyl-methyl, Benzyloxy, Phenoxy.

Verbindungen, in welchen
$R^1$ Wasserstoff, Methoxy oder Chlor,
n 1 oder 2,
$R^2$ Wasserstoff oder Phenyl und
$R^3$ unsubstituiertes Phenyl oder mit Nitro, Trifluormethyl, oder Amino mono-substituiertes Phenyl, mit Methoxy trisubstituiertes Phenyl oder mit Methylendioxy in 3.4-Stellung substituiertes Phenyl, Thienyl, Alkyl mit 1 bis 3 C-Atomen, Methoxy, 2-Hydroxy-3-methyl-propyloxy, Alkenyloxy mit 4 C-Atomen, Cyclohexyl, Benzyl, Diphenylmethyl oder Benzyloxy
bedeuten, sind besonders bevorzugt.

Die Verbindungen besitzen wertvolle pharmakologische, insbesondere Herz-Kreislauf-wirksame, Eigenschaften und sind als Arzneimittel geeignet. Die Erfindung betrifft

0007525



daher auch Arzneimittel auf Basis dieser Verbindungen sowie ihre Verwendung bei der Behandlung von Herz-Kreislauf-Krankheiten.

Das Verfahren zur Herstellung von Verbindungen der Formel I ist dadurch gekennzeichnet, daß man

a) ein 2-Alkoxy- oder 2-Alkylthio-3,4-dihydrochinolin der Formel II

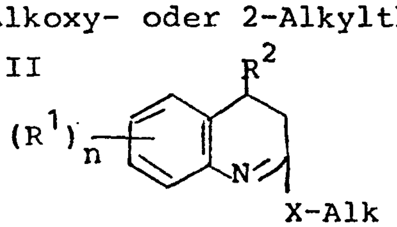

II

worin $R^1$, $R^2$ und n die zur Formel I genannte Bedeutung haben, X Sauerstoff oder Schwefel und Alk $C_1$-$C_3$-Alkyl, vorzugsweise Methyl oder Äthyl, bedeuten, mit einem 4-Aminopiperidin der Formel III,

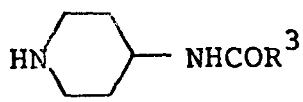

III

worin $R^3$ die zur Formel I genannte Bedeutung hat, umsetzt, oder

b) eine Verbindung der Formel IV

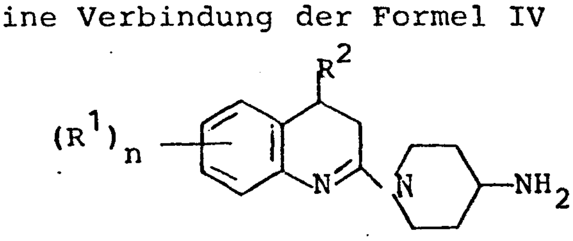

IV

worin $R^1$, $R^2$ und n die zur Formel I genannte Bedeutung haben, mit einer Carbonsäure der Formel HOOC-$R^3$ bzw. einem Carbonsäurehalogenid bzw. Halogenkohlensäurederivat der Formel Hal-CO-$R^3$ oder einem Carbonsäureanhydrid der Formel $(R^3CO)_2O$, worin $R^3$ die zur Formel I genannte Bedeutung hat und Hal ein Halogenatom bedeutet, umsetzt,
gegebenenfalls eine erhaltene Verbindung, worin $R^3$ einen $C_2$-$C_6$-Alkenylrest bedeutet, in bekannter Weise in eine Verbindung der Formel I, worin $R^3$

einen mit Hydroxy substituierten $C_2$-$C_6$ Alkoxyrest darstellt, überführt und gegebenenfalls die Verbindungen in ihre physiologisch verträglichen Salze überführt.

Nach dem Verfahren a kann die Umsetzung eines 2-Alkylthio-3,4-dihydrochinolins der Formel II mit einem 4-Aminopiperidin der Formel III in Abwesenheit eines Lösungsmittels vorgenommen werden. Werden Lösungsmittel verwendet, so kommen beispielsweise Äther wie Dioxan oder Diglym, aromatische Kohlenwasserstoffe wie Toluol oder Chlorbenzol, Alkohole wie Äthanol oder Isoamylalkohol, aprotische Lösungsmittel wie Dimethylformamid, Dimethylacetamid oder dergleichen in Frage. Die Reaktion wird bei einer Temperatur im Bereich von 50 bis 200°C, vorzugsweise zwischen 60 und 180°C unter Verwendung von vorzugsweise äquimolaren Mengen des Amins der Formel III ausgeführt.

Für die Umsetzung der 2-Alkoxy-3,4-dihydrochinoline mit Aminopiperidinen III werden im Prinzip die beschriebenen Reaktionsbedingungen angewendet, jedoch sind im allgemeinen im Vergleich zur Reaktion der 2-Alkylthioverbindungen etwas höhere Reaktionstemperaturen und längere Reaktionszeiten erforderlich.

Die Ausgangsverbindungen IV des Verfahrens b sind neu. Sie können nach Verfahren a) aus den Verbindungen der Formel II und 4-Aminopiperidin hergestellt werden. Vorzugsweise wird jedoch zunächst mit einem geschützten 4-Aminopiperidin umgesetzt. Als Schutzgruppe der 4-Aminofunktion können die aus der Peptidchemie bekannten Aminoschutzgruppen, z.B. die tert.-Butyloxycarbonyl-, die p-Methoxybenzyloxycarbonyl-, die Trifluoracetyl-, die 2-Nitro-4-methoxyphenylsulfenylschutzgruppe udgl., verwendet werden. In den so erhaltenen Verbindungen der Formel IV a

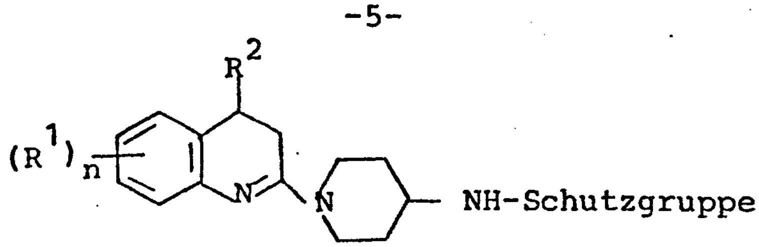

IV a

lassen sich die Schutzgruppen nach an sich bekannten Methoden unter Bildung der Verbindungen IV abspalten. Stellt die Schutzgruppe einen Rest der Formel $-COR^3$ dar, so ist eine Abspaltung nur dann erforderlich, wenn ein anderer Rest-$COR^3$ eingeführt werden soll. Vorzugsweise wird die tert. Butyloxycarbonylschutzgruppe verwendet, die quantitativ, z.B. mit Salzsäure/Eisessig oder Trifluoressigsäure unter Bildung der Verbindung IV abgespalten werden kann.

Die Acylierung der Verbindungen IV unter Bildung der Verbindungen I erfolgt nach an sich bekannten Methoden durch Reaktion mit den obengenannten Carbonsäuren HOOC-$R^3$, z.B, in Anwesenheit eines wasserabspaltenden Mittels wie Dicyclohexylcarbodiimd, bzw. mit den oben genannten Säurehalogeniden, Säureanhydriden oder Chlorkohlensäureestern der Formel Cl-CO-$R^3$.

Die Verbindungen der allgemeinen Formel I werden in freier Form oder als Salze isoliert, je nach den angewandten Reaktionsbedingungen. Die freien Basen können nach Reaktion mit anorganischen oder organischen Säuren in ihre pharmakologisch verträglichen Salze übergeführt werden. Solche Säuren sind z.B. Salzsäure, Schwefelsäure, Phosphorsäure, aliphatische, alicyclische, araliphatische, aromatische oder heterocyclische Carbonsäuren oder Sulfonsäuren wie Essigsäure, Weinsäure, Milchsäure, Maleinsäure, Fumarsäure, Zitronensäure, Oxalsäure, Methansulfonsäure, Hydroxyäthansulfonsäure oder synthetische Harze, die saure Gruppen enthalten.

Die erfindungsgemäßen Verbindungen sind für die Verwendung als Arzneimittel geeignet. Sie zeigen insbesondere blutdrucksenkende und antiarrythmische Eigenschaften. Diese Eigenschaften zeigen auch die Zwischenprodukte der Formel IV.

1) Die blutdrucksenkende Wirkung wurde am narkosierten Hund und an der Hochdruckratete bestimmt.

Hunde werden mit Pentobarbital-Natrium ((R) Nembutal) (35 mg/kg i.p.) narkotisiert, die Prüfsubstanz in Dosen von 0,5-5 mg/kg intravenös appliziert und die Blutdrucksenkung laufend über 30 bis 60 Minuten, bei längerer Wirksamkeit auch länger, fortlaufend registriert. Eine signifikante Senkung von mindestens 20 mm über mindestens 30 Minuten tritt z.B. nach Applikation der Verbindungen der Beispiele Nr. 6 und 31b in Dosen von jeweils 1 mg/kg ein.

Zur Prüfung an der genetischen Hochdruckratte werden die Substanzen oral verabreicht. Die Verbindung des Beispiels 31b bewirkt bei einer Dosierung von 15 mg/kg per os innerhalb von 18 Stunden einen Blutdruckabfall von 30 - 50 mm.

2) Zur Prüfung auf antiarrythmische Wirkung wird der mit Strophantin vergiftete Hund verwendet (Methode: Laszlo Szekeres, Institute of Pharmacology, University Medical School of Szeged, Hungary, "Methods for Evaluating Antiarrhytmic Agents" in Methods in Pharmacology I, New York 1971, Herausgeber A. Schwartz).

Hierfür wurden Bastard-Hunde beiderlei Geschlechts in Nembutal-Narkose (35 mg(kg i.p.) mit 4 µg/kg/ml/Min. K-Strophanthin per intravenöser Dauerinfusion bis zum Auftreten gehäufter ventrikulärer Extrasystolen vergiftet. Die Prüfsubstanzen wurden intravenös in 5 % wässriger Lösung verabreicht. Registrierung des EKG (Extremitätenableitungen) und des Blutdrucks auf einem Direktschreiber, gleichzeitige Beobachtung auf einem Oscilloskop. Als wirksam gewertet wurde eine Substanz, die die genannten

Rhythmusstörungen völlig zu beseitigen vermochte. Die Verbindungen der Beispiele 31a und 34 bewirken dies nach Verabreichung einer Dosis von 1 mg/kg.

Die neuen Verbindungen können entweder allein oder mit physiologisch verträglichen Hilfs- oder Trägerstoffen vermischt angewandt werden. Sie können oral, parenteral, i.m. oder i.v. verabreicht werden. Für eine orale Anwendungsform werden die aktiven Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige, alkoholische oder ölige Lösungen. Als inerte Träger können z,B, Magnesiumcarbonat, Milchzucker oder Maisstärke unter Zusatz anderer Stoffe wie z.B. Magnesiumstearat verwendet werden. Dabei kann die Zubereitung als Trocken- oder Feuchtgranulat erfolgen. Als ölige Trägerstoffe oder Lösungsmittel kommen besonders pflanzliche oder tierische Öle in Betracht wie z.B. Sonnenblumenöl oder Lebertran.

Als Lösungsmittel der entsprechenden physiologisch verträglichen Salze der aktiven Verbindungen für eine intravenöse Applikation kommen z. B. in Frage: Wasser, physiologische Kochsalzlösung oder Alkohole wie z.B. Äthanol, Propandiol oder Glycerin, daneben auch Zuckerlösungen wie z.B. Glukose- oder Mannitlösungen, oder auch eine Mischung aus den verschiedenen genannten Lösungsmitteln.

Die erfindungsgemäße Verbindungen sind innerhalb eines breiten Dosierungsbereiches wirksam. Die Menge Wirksubstanz pro Dosierungseinheit bzw. die täglich zu verabreichende Dosis kann daher ebenfalls variieren. Sie hängt ab von der Art der Applikation, vom Zustand, Gewicht usw. des zu Behandelnden. Die tägliche Dosierung bei oraler Applikation liegt vorzugsweise zwischen 60 und 1000 mg Wirksubstanz, insbesondere zwischen 100 und 500 mg.

Eine Dosierungseinheit wie z.B. eine Tablette enthält vorzugsweise 50 bis 250 mg. Für die i.v. oder i.m. Anwendung beträgt die tägliche Dosierung vorzugsweise 10 bis 200 mg, insbesondere 20 bis 100 mg.

Die Erfindung wird durch die folgenden Beispiele näher erläutert, jedoch nicht beschränkt.

Beispiel 1

2-(4-Benzamido-1-piperidyl)-3,4-dihydrochinolin

Ein Gemisch aus 3,6 g (0,02 Mol) 2-Methylthio-3,4-dihydrochinolin und 4 g (0,02 Mol) 4-Benzamidopiperidin wird in einem Bad von 140°C erwärmt. Die Entwicklung von Methylthiol ist nach 10 Minuten praktisch beendet, nach 15 Minuten erstarrt die Schmelze zu einer kristallinen Masse. Es wird mit 40 ml Äthylacetat verrieben, abgesaugt und mit Äthylacetat gewaschen.

Ausbeute: 5,6 g (85 % d.Th.), Schmp. 202°C.
3,3 g (0,01 Mol) werden in 30 ml Methylendichlorid gelöst und ein geringer Überschuß äthanolischer Salzsäure zugegeben. Es bildet sich ein Niederschlag, der abgesaugt und mit Methylendichlorid gewaschen wird.
Ausbeute: 3,2 g (87 % d.Th.) Hydrochlorid, Schmp. 278°C / Zersetzung

Beispiel 2

4-Phenyl-2-/¯4-(3,4,5-trimethoxybenzamido)-1-piperidyl 7-3,4-dihydrochinolin

3 g (0,012 Mol) 2-Äthoxy-4-phenyl-3,4-dihydrochinolin und 3,5 g (0,012 Mol) 4-(3,4,5-Trimethoxy)benzamido-piperidin werden in 25 ml Diglym 16 Stunden unter Rückfluß gekocht. Das Lösungsmittel wird im Vakuum entfernt, der kristalline Rückstand mit Äther digeriert, abgesaugt und mit Äther gewaschen.
Ausbeute: 4,2 g (70 % d.Th.) , Schmp. 117°C

Das Hydrochlorid wird wie vorstehend hergestellt. Ausbeute quantitativ, Schmp. 265 - 266°C unter Zersetzung.

Die Verbindungen der folgenden Tabelle werden in Analogie zu den Beispielen 1 und 2 nach den dort angegebenen Verfahren hergestellt.

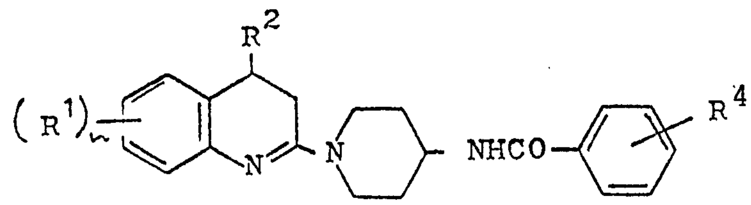

| Beispiel-Nr. | $R^1$ | $R^2$ | R 4 | Base Schmp. | Hydrochlorid °C |
|---|---|---|---|---|---|
| 3 | H | H | 3,4,5-tri-$CH_3O$ | 206-208 | 248-250 |
| 4 | H | H | 3-$CF_3$ | 178 | 284 |
| 5 | H | H | 3-$NO_2$ | 176 | 275-277 |
| 6 | 6,7-di-$CH_3O$ | H | H | amorph | 260-262 |
| 7 | 6,7-di-$CH_3O$ | H | 3,4,5-tri-$CH_3O$ | amorph | 171-173 |
| 8 | H | $C_6H_5$ | H | amorph | 250-251 |
| 9 | H | $C_6H_5$ | 2,4,5-tri-$CH_3O$ | Harz | 198-200 |
| 10 | H | $C_6H_5$ | 3,4-Methylendioxy | Harz | 158-160 |
| 11 | H | $C_6H_5$ | 3-$CF_3$ | 194-195 | 285-286 |
| 12 | H | $C_6H_5$ | 3-$NO_2$ | 150 | 270-272 |
| 13 | 6-$CH_3O$ | $C_6H_5$ | H | 156-158 | 240-242 |
| 14 | 6-$CH_3O$ | $C_6H_5$ | 3,4,5-tri-$CH_3O$ | 180-182 | 235-237 |

| Beispiel-Nr. | $R^1$ | $R^2$ | R4 | Base Schmp. | Hydrochlorid °C |
|---|---|---|---|---|---|
| 15 | 6-$CH_3O$ | $C_6H_5$ | 2,4,5-tri-$CH_3O$ | 170-172 | 191-193 |
| 16 | 6-$CH_3O$ | $C_6H_5$ | 3,4-Methylendioxy | Harz | 198-200 |
| 17 | 6-$CH_3O$ | $C_6H_5$ | 3-$CF_3$ | 128 | 230 |
| 18 | 6-$CH_3O$ | $C_6H_5$ | 3-$NO_2$ | Harz | 262-264 |
| 19 | 6,7-di-$CH_3O$ | $C_6H_5$ | H | amorph | 178-180 |
| 20 | 6,7-di-$CH_3O$ | $C_6H_5$ | 3,4,5-tri-$CH_3O$ | 198-200 | 166-168 |
| 21 | 6,7-di-$CH_3O$ | $C_6H_5$ | 2,4,5-tri-$CH_3O$ | Harz | 198-200 |
| 22 | 6,7-di-$CH_3O$ | $C_6H_5$ | 3,4-Methylendioxy | 140-142 | 186-188 |
| 23 | 6,7-di-$CH_3O$ | $C_6H_5$ | 3-$CF_3$ | 142 | 186-189 |
| 24 | 6,7-di-$CH_3O$ | $C_6H_5$ | 3-$NO_2$ | Harz | 205-207 |
| 25 | 6,8-di-$CH_3O$ | $C_6H_5$ | H | Harz | 164-166 |
| 26 | 6,8-di-$CH_3O$ | $C_6H_5$ | 3,4,5-tri-$CH_3O$ | Harz | 144-146 |
| 27 | 6,8-di-$CH_3O$ | $C_6H_5$ | 3,4-Methylendioxy | Harz | 161 |
| 28 | 6-Cl | $C_6H_5$ | H | amorph | 297 |
| 29 | 6-Cl | $C_6H_5$ | 3,4,5-tri-$CH_3O$ | 224-225 | 245-247 |

Beispiel 30

2-/¯4-(3-Aminobenzamido)-1-piperidyl 7-3,4-dihydrochinolin

2,1 g (5 mMol) 2-/¯4-(3-Nitrobenzamido)-1-piperidyl_7-3,4-dihydrochinolin-Hydrochlorid (Beispiel 5) werden in 40 ml Methanol gelöst und nach Zugabe von 0,5 g Raney-Nickel in einer Schüttelapparatur bei Raumtemperatur hydriert. Nach beendeter Wasserstoffaufnahme wird vom Katalysator abgesaugt, das Lösungsmittel im Vakuum entfernt, der kristalline Rückstand mit Aceton digeriert, abgesaugt und mit Aceton gewaschen.
Ausbeute: 1,8 g (93 % d.Th.) Monohydrochlorid, Schmp. 245 - 247 °C unter Zersetzung.

Beispiel 31

2-/¯4-(2-Thenoylamido)-1-piperidyl 7-3,4-dihydrochinolin

a) 2-(4-Tert.butyloxycarbonylamino-1-piperidyl)-3,4-dihydrochinolin

Ein Gemisch aus 5,3 g (0,03 Mol) 2-Methylthio-3,4-dihydrochinolin und 6 g (0,03 Mol) 4-Tert.butyloxycarbonylamino-piperidin wird 4 Stunden auf 140° erhitzt. Der kristalline erkaltete Rückstand wird mit Äther verrieben, abgesaugt und mit Äther gewaschen.
Ausbeute: 8,9 g (90 % d.Th.), Schmp. 136°C.

b) 2-(4-Amino-1-piperidyl)-3,4-dihydrochinolin

6,6 g (0,02 Mol) 2-(4-Tert.butyloxycarbonylamino-1-piperidyl)-3,4-dihydrochinolin werden in 50 ml Trifluoressigsäure gelöst. Durch Kühlen wird die Temperatur zwischen 30 und 35°C gehalten. Nach 30 Minuten wird überschüssige Trifluoressigsäure im Vakuum entfernt, der Rückstand mit Eis und 4N Natronlauge behandelt und

4 x mit Methylendichlorid extrahiert. Nach Entfernung des Lösungsmittels verbleibt die Titelverbindung als Harz.
Ausbeute: 4,1 g ( 90 % d.Th.)
Das Dihydrochlorid wird in Analogie zu Beispiel 1 hergestellt
Schmp.133- 35 °C unter Zersetzung

c) 2-/¯4-(2-Thenoylamido)-1-piperidyl_7-3,4-dihydrochinolin

Zu einer Lösung von 3,6 g (0,016 Mol) 2-(4-Amino-1-piperidyl)-3,4-dihydrochinolin in 40 ml Chloroform und 10 ml Pyridin wird unter Kühlung eine Lösung von 0,024 Mol 2-Thenoylchlorid in 15 ml Chloroform gegeben. Die Mischung wird 3 Stunden bei Raumtemperatur belassen, mit Chloroform verdünnt, mit Wasser gewaschen und getrocknet.

Das Lösungsmittel wird im Vakuum entfernt, der harzige Rückstand in Aceton gelöst und mit äthanolischer Salzsäure angesäuert. Nach Verdampfen des Lösungsmittels verbleibt ein Harz, das in 30 ml Aceton gelöst wird. Nach Anreiben bildet sich ein farbloser Niederschlag, der nach 3 Stunden abgesaugt, mit Aceton gewaschen und getrocknet wird.

Ausbeute 4,2 g /70 % d.Th.) Hydrochlorid,
Schmp. 215-217°C unter Zersetzung

Beispiel 32

2-(4-Cyclohexan-carbonylamino-1-piperidyl)-3',4-dihydro-chinolin

Ein Gemisch aus 5,3 g (0,03 Mol) 2-Methylthio-3,4-dihydrochinolin und 6,3 g (0,03 Mol) 4-Cyclohexancarbonylamino-piperidin wird 4 Stunden auf 140°C erhitzt. Der kristalline erkaltete Rückstand (Titelverbindung) wird mit Äther verrieben, abgesaugt und mit Äther gewaschen.
Base: Ausbeute 2,6 g (77 % d.Th.), Schmp. 173 - 175°C.
Hydrochlorid Schmp. 260 - 262°C unter Zersetzung

Die Verbindungen der folgenden Beispiele werden in Analogie zu Beispiel 32 aus 2-Methylthio-3,4-dihydrochinolin und den entsprechenden Piperidinderivaten 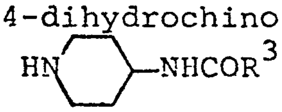 dargestellt.

Beispiel 33

2-(4-Phenylacetyl-amino-1-piperidyl)-3,4-dihydrochinolin

Base: Ausbeute 55 % d.Th., Schmp. 169 - 170°C
Hydrochlorid: Schmp. > 140°C unter Zersetzung

Beispiel 34

2-(4-Butyryl-amino-1-piperidyl)-3,4-dihydrochinolin

Base: Ausbeute 77 % d.Th., Schmp. 113 - 115°C
Hydrochlorid: Schmp. 239 - 241°C unter Zersetzung

Beispiel 35

2-(4-Methoxy-carbonylamino-1-piperidyl)-3,4-dihydro-chinolin

Ein Gemisch aus 2,29 g (0,01 Mol) 2-(4-Amino-1-piperidyl)-3,4-dihydrochinolin, 1,9 g (0,02 Mol) Chlorameisensäuremethylester, 10 g Kaliumcarbonat und 2,5 ml Toluol wird 18 Stunden bei Raumtemperatur gerührt.

Nach Zugabe von 100 ml Methylendichlorid wird 4 x mit Wasser gewaschen und das Lösungsmittel im Vakuum entfernt. Der ölige Rückstand der organischen Phase wird in Aceton gelöst, mit einem geringen Überschuß an äthanolischer Salzsäure versetzt und zweimal mit Aceton abgedampft. Der kristalline Rückstand (Titelverbindung) wird mit Aceton aufgekocht, abgesaugt und mit Aceton gewaschen.
Ausbeute an Hydrochlorid: 2,5 g (78 % d.Th.)
Schmp. 231 - 232°C unter Zersetzung.

Die Verbindungen der folgenden Beispiele werden in Analogie zu Beispiel 35 aus 2-(4-Amino-1-piperidyl)-3,4-dihydrochinolin und der entsprechenden Verbindung $ClCOR^3$ dargestellt.

Beispiel 36

2-/4-(2-Methyl-2-propenyl)oxycarbonylamino-1-piperidyl_7-3,4-dihydrochinolin

Base: Öl, Ausbeute: 60 % d. Th.
Hydrochlorid: Schmp. 225 - 227°C

Beispiel 37

2-(4-Benzyloxycarbonylamino-1-piperidyl)-3,4-dihydrochinolin

Base: Öl, Ausbeute: 65 % d. Th.
Hydrochlorid: Schmp. 172 - 174°C.

Beispiel 38

2-(4-Diphenylacetylamino-1-piperidyl)3,4-dihydrochinolin

Base: Harz, Ausbeute: 52 % d.Th.
Hydrochlorid: Schmp. 260 - 262°C unter Zersetzung.

Beispiel 39

2-/4-(2-Hydroxy-2-methylpropyl)oxycarbonyl-amino-1-piperidyl_7-3,4-dihydrochinolin

3,8 g (0,01 Mol) 2-/4-(2-Methyl-2-propenyl)oxycarbonyl-amino-1-piperidyl_7-3,4-dihydrochinolin (Beispiel 5) werden in 30 ml 60 prozentiger Schwefelsäure gelöst und 18 Stunden bei Raumtemperatur belassen. Die Lösung wird mit konzentrierter wässriger Sodalösung alkalisch gestellt und 4 x mit je 50 ml Methylendichlorid extrahiert. Der Rückstand der organischen Phase wird über Kieselgel 60 (0,063 - 0,2 mm, Merck, desaktiviert mit 10 % Wasser) mit Chloroform: Methanol: konz. Ammoniak (100:10:1 )chromatographiert. Die Titelverbindung wird als Harz eluiert. Ausbeute 2 g (58 % d.Th.). Das Hydrochlorid schmilzt bei 212 - 213°C.

Patentansprüche:

1. Verbindungen der Formel I

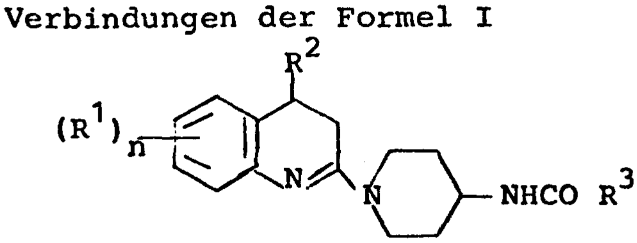

worin bedeuten:

n eine Zahl von 1 bis 3

$R^1$ gleiche oder verschiedene Reste der Bedeutung: Wasserstoff, $C_1$-$C_6$-Alkyl, $C_1$-$C_6$-Alkoxy, Benzyloxy, Methylendioxy, Halogen, Trifluormethyl, Hydroxy, Nitro, Amino, $C_1$-$C_6$-Alkoxycarbonyl, Sulfamoyl,

$R^2$ Wasserstoff, Phenyl, Benzyl, $C_1$-$C_4$-Alkyl und

$R^3$ $C_1$-$C_6$-Alkyl, $C_2$-$C_6$-Alkenyl, $C_1$-$C_6$-Alkoxy, das gegebenenfalls mit Hydroxy substituiert ist, $C_2$-$C_6$-Alkenyloxy, Cycloalkyl mit 5 bis 7 Kohlenstoffatomen, Cycloalkoxy mit 5 bis 7 Kohlenstoffatomen, Aryl, Aryl-$C_1$-$C_6$-alkyl, Diaryl-$C_1$-$C_6$-alkyl, Aryl-$C_1$-$C_6$-alkyloxy oder Aryloxy, wobei Aryl für Phenyl, Furyl, Thienyl oder Pyridyl steht und wobei die Arylgruppen mono-, di- oder trisubstituiert sein können mit $C_1$-$C_6$-Alkyl, $C_1$-$C_6$-Alkoxy, Benzyloxy, Methylendioxy, Halogen, Trifluormethyl, Hydroxy, Nitro, Amino, $C_1$-$C_6$ Alkoxy-carbonyl oder Sulfamoyl

sowie deren physiologisch verträgliche Salze.

2. Verbindungen der Formel IV

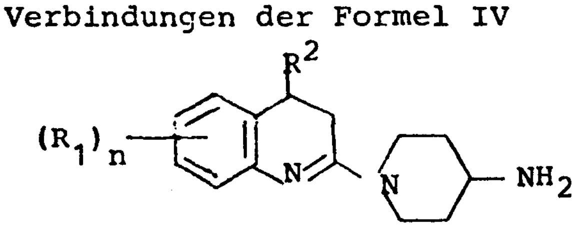

worin $R^1$, n und $R^2$ die zur Formel I in Anspruch 1 genannte Bedeutung haben.

3. Verfahren zur Herstellung von Verbindungen der Formel I, dadurch gekennzeichnet, daß man

a) ein 2-Alkoxy- oder 2-Alkylthio-3,4-dihydrochinolin der Formel II

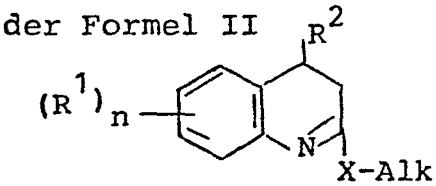

II

worin $R^1$, $R^2$ und n die zur Formel I genannte Bedeutung haben, X Sauerstoff oder Schwefel und Alk $C_1$-$C_3$-Alkyl bedeuten, mit einem 4-Aminopiperidin der Formel III

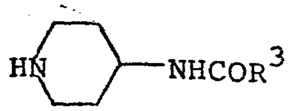

III

worin $R^3$ die zur Formel I genannte Bedeutung hat, umsetzt, oder

b) eine Verbindung der Formel IV

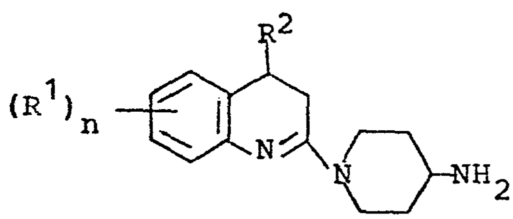

IV

worin $R^1$, $R^2$ und n die zur Formel I genannte Bedeutung haben mit einer Carbonsäure der Formel HOOC-$R^3$ bzw. einem Carbonsäurehalogenid bzw. Halogenkohlensäurederivat der Formel Hal-CO-$R^3$ oder einem Carbonsäureanhydrid der Formel $(R^3\text{-}CO)_2O$, worin $R^3$ die zur Formel I genannte Bedeutung hat und Hal ein Halogenatom bedeutet, umsetzt, gegebenenfalls eine erhaltene Verbindung, worin $R^3$ einen $C_2$-$C_6$ Alkenylrest bedeutet, in bekannter Weise in eine Verbindung der Formel I, worin $R^3$ einen mit Hydroxy substituierten $C_2$-$C_6$ Alkoxyrest darstellt, überführt und gegebenenfalls die Verbindungen in ihre physiologisch verträglichen Salze überführt.

4. Verfahren zur Herstellung von Arzneimitteln, dadurch gekennzeichnet, daß man eine Verbindung gemäß Anspruch 1, gegebenenfalls mit üblichen pharmazeutischen Trägern und/oder Stabilisatoren, in eine therapeutisch geeignete Darreichungsform bringt.

5. Arzneimittel, gekennzeichnet durch einen Gehalt an einer Verbindung gemäß Anspruch 1 oder bestehend aus einer solchen Verbindung.

6. Verwendung einer Verbindung gemäß Anspruch 1 zur Behandlung von Herz-Kreislauf-Krankheiten.

7. Verfahren zur Behandlung von Herz-Kreislauf-Erkrankungen, dadurch gekennzeichnet, daß man eine wirksame Menge einer Verbindung gemäß Anspruch 1 verabreicht.

Patentansprüche für Österreich:

1. Verfahren zur Herstellung von Verbindungen der Formel I

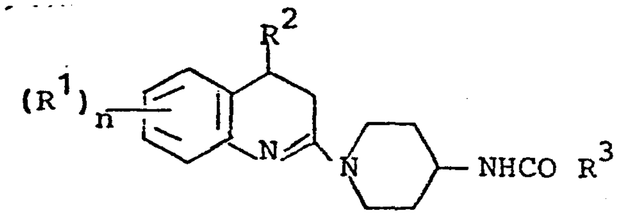

I

worin bedeuten:

n eine Zahl von 1 bis 3

$R^1$ gleiche oder verschiedene Reste der Bedeutung: Wasserstoff, $C_1$-$C_6$-Alkyl, $C_1$-$C_6$-Alkoxy, Benzyloxy, Methylendioxy, Halogen, Trifluormethyl, Hydroxy, Nitro, Amino, $C_1$-$C_6$-Alkoxycarbonyl, Sulfamoyl,

$R^2$ Wasserstoff, Phenyl, Benzyl, $C_1$-$C_4$-Alkyl und

$R^3$ $C_1$-$C_6$-Alkyl, $C_2$-$C_6$-Alkenyl, $C_1$-$C_6$-Alkoxy, das gegebenenfalls mit Hydroxy substituiert ist, $C_2$-$C_6$-Alkenyloxy, Cycloalkyl mit 5 bis 7 Kohlenstoffatomen, Cycloalkoxy mit 5 bis 7 Kohlenstoffatomen, Aryl, Aryl-$C_1$-$C_6$-alkyl, Diaryl-$C_1$-$C_6$-alkyl, Aryl-$C_1$-$C_6$-alkyloxy oder Aryloxy, wobei Aryl für Phenyl, Furyl, Thienyl oder Pyridyl steht und wobei die Arylgruppen mono-, di- oder trisubstituiert sein können mit $C_1$-$C_6$-Alkyl, $C_1$-$C_6$-Alkoxy, Benzyloxy, Methylendioxy, Halogen, Trifluormethyl, Hydroxy, Nitro, Amino, $C_1$-$C_6$ Alkoxy-carbonyl oder Sulfamoyl

sowie deren physiologisch verträglichen Salzen,

dadurch gekennzeichnet, daß man

a) ein 2-Alkoxy- oder 2-Alkylthio-3,4-dihydrochinolin der Formel II

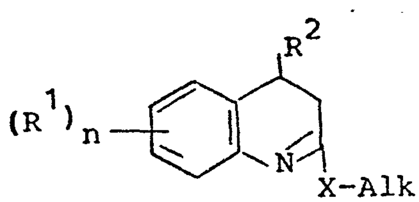

II

worin $R^1$, $R^2$ und n die zur Formel I genannte Bedeutung haben, X Sauerstoff oder Schwefel und Alk

$C_1$-$C_3$-Alkyl bedeuten, mit einem 4-Aminopiperidin der Formel III

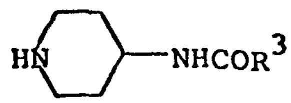

III

worin $R^3$ die zur Formel I genannte Bedeutung hat, umsetzt, oder

b) eine Verbindung der Formel IV

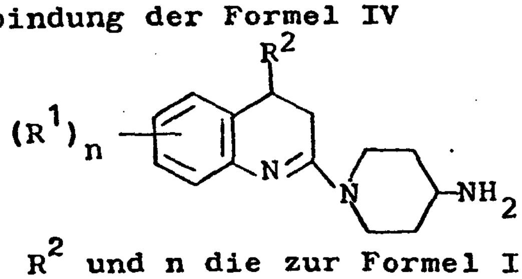

IV

worin $R^1$, $R^2$ und n die zur Formel I genannte Bedeutung haben mit einer Carbonsäure der Formel HOOC-$R^3$ bzw. einem Carbonsäurehalogenid bzw. Halogenkohlensäurederivat der Formel Hal-CO-$R^3$ oder einem Carbonsäureanhydrid der Formel $(R^3\text{-CO})_2O$, worin $R^3$ die zur Formel I genannte Bedeutung hat und Hal ein Halogenatom bedeutet, umsetzt,

gegebenenfalls eine erhaltene Verbindung, worin $R^3$ einen $C_2$-$C_6$ Alkenylrest bedeutet, in bekannter Weise in eine Verbindung der Formel I, worin $R^3$ einen mit Hydroxy substituierten $C_2$-$C_6$ Alkoxyrest darstellt, überführt und gegebenenfalls die Verbindungen in ihre physiologisch verträglichen Salze überführt.

2. Verfahren nach Anspruch 1, d.h., daß Verbindungen der Formel IV

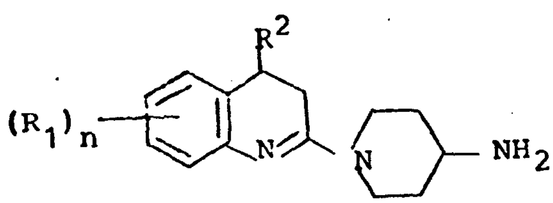

worin $R^1$, n und $R^2$ die zur Formel I in Anspruch 1 genannte Bedeutung haben, hergestellt werden.

3. Verfahren zur Herstellung von Arzneimitteln, dadurch gekennzeichnet, daß man eine Verbindung gemäß Anspruch 1,

gegebenenfalls mit üblichen pharmazeutischen Trägern und/oder Stabilisatoren, in eine therapeutisch geeignete Darreichungsform bringt.

4. Arzneimittel, gekennzeichnet durch einen Gehalt an einer Verbindung gemäß Anspruch 1 oder bestehend aus einer solchen Verbindung.

5. Verwendung einer Verbindung gemäß Anspruch 1 zur Behandlung von Herz-Kreislauf-Krankheiten.

6. Verfahren zur Behandlung von Herz-Kreislauf-Erkrankungen, dadurch gekennzeichnet, daß man eine wirksame Menge einer Verbindung gemäß Anspruch 1 verabreicht.

Europäisches Patentamt

**EUROPÄISCHER TEILRECHERCHENBERICHT, der nach Regel 45 des Europäischen Patentübereinkommens für das weitere Verfahren als europäischer Recherchenbericht gilt**

Nummer der Anmeldung
EP 79 102 395.5

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl.3) |
|---|---|---|---|
| | US - A - 3 538 101 (CIBA CORP.)<br>* Spalte 3, Zeilen 24 bis 62 *<br>-- | 3 | C 07 D 401/04<br>C 07 D 405/14<br>C 07 D 409/14<br>A 61 K 31/445<br>A 61 K 31/47 |
| | US - A - 3 647 802 (CIBA CORP.)<br>* Spalte 3, Zeile 46 bis Spalte 4, Zeile 17 *<br>---- | 3 | |

RECHERCHIERTE SACHGEBIETE (Int. Cl.3)

A 61 K 31/445
A 61 K 31/47
C 07 D 401/04
C 07 D 405/14
C 07 D 409/14

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5
Unvollständig recherchierte Patentansprüche: -
Nicht recherchierte Patentansprüche: 6,7
Grund für die Beschränkung der Recherche:

Verfahren zur therapeutischen Behandlung des menschlichen oder tierischen Körpers

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 15-11-1979 | FROELICH |